# EUROPÄISCHE PATENTSCHRIFT

(11) **EP 0 555 769 B1**
(45) Veröffentlichungstag und Bekanntmachung des Hinweises auf die Patenterteilung: **15.09.1999**
(21) Anmeldenummer: 93101722.2
(22) Anmeldetag: 04.02.1993
(51) Int. Cl.: C07D 501/34, C07D 501/04, C07D 277/20, C07D 501/00

(54) **Verfahren zur Herstellung von Cephem-Prodrug-Estern**
Process for the preparation of Cephem-Prodrug-esters
Procédé pour la préparation d'esters de céphem, utilisés comme Prodrug

(30) Priorität: 14.02.1992 DE 4204349
(43) Veröffentlichungstag der Anmeldung: 18.08.1993
(73) Patentinhaber: HOECHST AKTIENGESELLSCHAFT, 65926 Frankfurt am Main (DE)
(72) Erfinder: Defossa, Elisabeth, Dr., W-6270 Idstein/Ts (DE); Fischer, Gerd, Dr., W-6250 Limburg 8 (DE); Gerlach, Uwe, Dr., W-6230 Frankfurt/M (DE); Hörlein, Rolf, Dr., W-6000 Frankfurt/M (DE); Krass, Norbert, Dr., W-6000 Frankfurt/M (DE); Lattrell, Rudolf, Dr., W-6240 Königstein/Ts (DE); Stache, Ulrich, Dr., W-6238 Hofheim/Ts (DE); Wollmann, Theodor, Dr., W-6238 Hofheim/Ts (DE)

(56) Entgegenhaltungen:
- EP-A- 0 049 119
- EP-A- 0 134 420
- EP-A- 0 329 008
- EP-A- 0 334 281
- EP-A- 0 355 821
- EP-A- 0 379 132
- EP-A- 0 402 806
- WO-A-92/07840

## Beschreibung

Gegenstand der Erfindung ist ein Verfahren zur Herstellung von Cephem-Prodrug-Estern der Formel 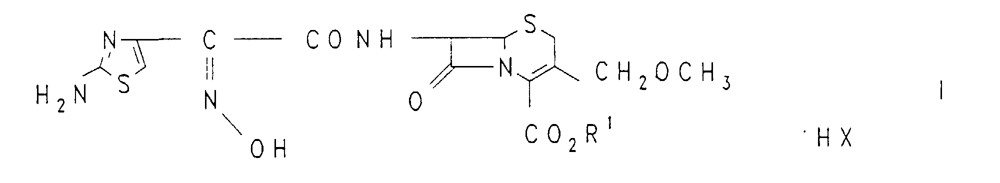 worin R¹ C₁-C₅-Alkanoyloxy-C₁-C₃-alkyl oder C₁-C₅-Alkoxycarbonyloxy-C₁-C₃-alkyl und H X eine anorganische oder organische Säure ausgewählt aus: HCI, HBr oder H₂SO₄ und Methan-, Ethan-, Benzol-, p-Toluol-, p-Chlorobenzol- oder 2,4-Dichlorobenzolsulfonsäure, bedeutet, und worin die Hydroxyiminogruppe in der syn-Form vorliegt, wobei man eine Verbindung der Formel II 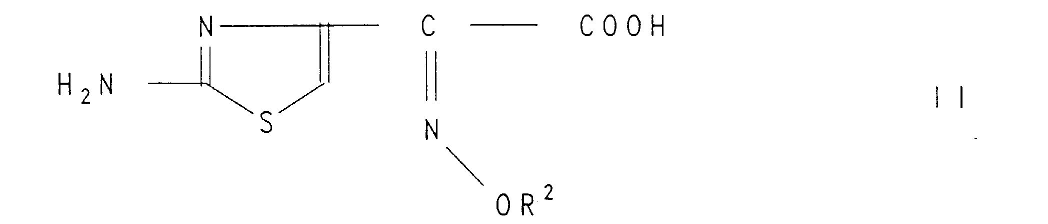 worin
R² eine durch saure Hydrolyse abspaltbare Schutzgruppe bedeutet, mit einem Bis(benzthiazol-2-yl)disulfid der Formel VI 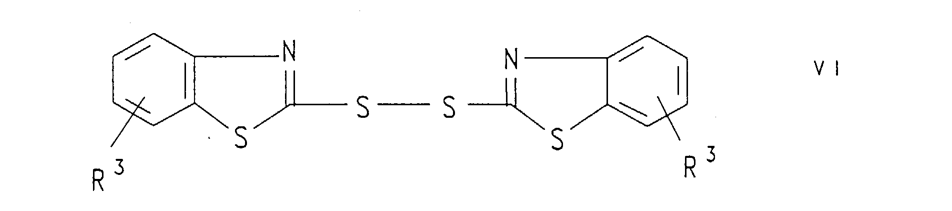 worin R³ Wasserstoff, C₁-C₆-Alkyl, C₂-C₆-Alkenyl, C₃-C₆-Alkinyl, C₁-C₆-Alkyloxy, C₂-C₆-Alkenyloxy, C₃-C₆-Alkinyloxy, Hydroxy, Acetoxy, Halogen, Nitro, Amino, Carboxy oder die Sulfogruppe bedeutet, und Triphenylphosphin in einem inerten Lösungsmittel, in Gegenwart eines tertiären Amins, zu einer Verbindung der Formel V 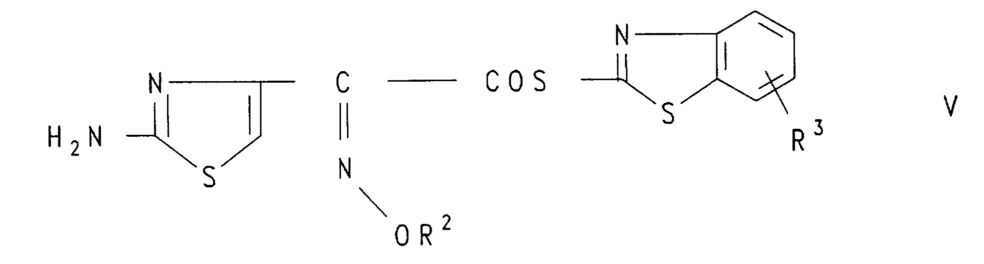 worin
R² und R³ die obengenannten Bedeutungen haben, reagieren läßt, anschließend diese Verbindung mit einem 7-Amino-ceph-3-em-4-carbonsäureester der Formel III 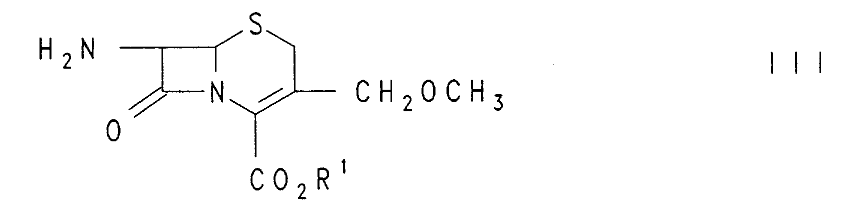 in der R¹ die obengenannte Bedeutung hat, in inerten organischen oder dipolar aprotischen Lösungsmitteln bei Temperaturen zwischen 0° und +80° C umsetzt und die Oxim-geschützte Verbindung der Formel IV, 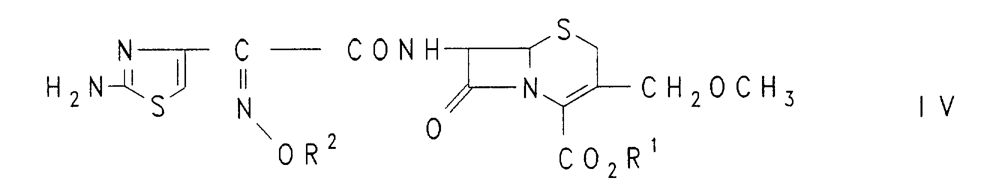 in der R¹ und R² die oben genannten Bedeutungen besitzen und worin die geschützte Oximgruppe in der syn-Form vorliegt bildete.

EP 402 806 beschreibt ein Verfahren zur Herstellung von kristallinen Cephem Säureadditionssalzen wobei Cephemcarbonsäure mit Benzolsulfonsäure, die gegebenenfalls mit C₁-C₄-Alkyl substituiert ist, umgesetzt wird, um das entsprechende Salz zu bilden.

In EP 379 132, EP 329 008 und EP 334 281 sind Verfahren zur Herstellung von amorphen Cephemcarbonsäureestern und ihrer physiologisch verträglichen Säureadditionssalze beschrieben.

In EP 49 119 und EP 134 420 werden Verfahren zur Herstellung von Cephemcarbonsäureestern und ihrer pyhsiologisch verträglichen Säureadditionssalze beschrieben, die die Reaktion zwischen einem Cephemcarbonsäureester, der an Stellung 7 mit 2-Halo-acetyl-2-hydroxyimino acetamido substituiert ist, und Thioharnstoff umfaßt, wobei ein Thiazolring gebildet wird.

In WO 92/07840 sind die Zwischenprodukte (Z)-2-(2-Aminothiazol-4-yl)-2-trityloxyimino-Essigsäure und (Z)-2-(2-Aminothiazol-4-yl)-2-tetrahydropyran-2-yloxyimino-Essigsäure beschrieben.

Das erfindungsgemäße Verfahren ist dadurch gekennzeichnet, daß die Verbindung der obengenannten Formel IV mit anorganischen Säuren oder mit aliphatischen oder aromatischen Sulfonsäuren ausgewählt aus: HCI, HBr oder H₂SO₄ und Methan-, Ethan-, Benzol-, p-Toluol-, p-Chlorobenzol- oder 2,4-Dichlorobenzolsulfonsäure, in organischen Lösungsmitteln ausgewählt aus: Alkohole, Ester, Ether und Ketone bei Temperaturen zwischen +20° und +110° C unter Bildung der Verbindung der Formel I in einem Schritt behandelt wird und daß das Ausgangsprodukt der Formel II nicht NH₂ geschützt ist.

Als bevorzugt kommen die folgenden Reste in Betracht:
R¹ = Acetoxymethyl, Propionyloxymethyl, Isopropionyloxymethyl, N-Butyryloxymethyl, Isobutyryloxymethyl, 2,2-Dimethylpropionyloxymethyl, Isovaleryloxymethyl, 1-Acetoxy-1-ethyl, 1-Acetoxy-1-propyl, 2,2-Dimethylpropionyloxy-1-ethyl, 1-Methoxycarbonyloxy-1-ethyl, 1-Ethoxycarbonyloxy-1-ethyl, 1-Isopropoxycarbonyloxy-1-ethyl oder Methoxycarbonyloxymethyl;
R² = C(C₆H₅)₃, Tetrahydropyranyl oder 2-Methoxy-2-propyl;
R³ = Wasserstoff, Methyl, Ethyl, Propyl, Methoxy, Ethoxy, Propoxy, Isopropoxy oder Chlor und
X = Cl⁻, Br⁻, HSO₄⁻, CH₃SO₃⁻, C₂H₅SO₃⁻, C₆H₅SO₃⁻ oder p-CH₃-C₆H₄-SO₃⁻.

Besonders bevorzugt für R¹ ist 2,2-Dimethylpropionyloxy-1-ethyl, insbesondere in der Form der reinen (S)- bzw. (R)-Diastereomeren, ganz besonders in der Form des reinen (S)-Diastereomers.

Die Umsetzung der Verbindung der Formel II mit Bis(benzthiazol-2-yl)disulfid der Formel VI und Triphenylphosphin wird in einem inerten Lösungsmittel, vorzugsweise in Dichlormethan oder Essigsäureethylester, in Gegenwart eines tertiären Amins, wie z. B. Triethylamin oder Diisopropylethylamin, durchgeführt. Die Verbindung der Formel V mit R² = C(C₆H₅)₃, R³ = H wird bei Verwendung von 1 - 2 Molen Triethylamin in hoher Ausbeute und in sehr reiner Form erhalten. Das gleiche gilt für die Verbindung V mit R² = C(C₆H₅)₃ und R³ = 6-Ethoxy oder 5-Chlor.

Die Umsetzung der Verbindungen der Formel V mit der Aminocephemverbindung der Formel III wird in inerten organischen Lösungsmitteln wie Ethylacetat, Dichlormethan, Tetrahydrofuran oder dipolar aprotischen Lösungsmitteln wie Dimethylformamid, Dimethylacetamid oder Dimethylsulfoxid bei Temperaturen zwischen 0° und +80°, vorzugsweise zwischen 20° und 50°C, ausgeführt. Im Falle der Verbindung der Formel V mit R² = C(C₆H₅)₃ werden wegen deren Schwerlöslichkeit vorzugsweise dipolare aprotische Lösungsmittel verwendet. Die Reaktionsprodukte der Formel IV werden in diesem Falle in einfacher Weise durch Eingießen der Reaktionsmischung in Wasser, Absaugen des ausgefallenen Produkts und Trocknen, isoliert.

Die Ausgangsverbindungen der Formel III können sowohl Diastereomerengemische als auch reine (S)- oder (R)-Diastereomere sein, wobei z.B. die Verbindungen der Formel III mit R¹ = -CH(CH₃)OCOC(CH₃)₃ in der (S)- und (R)-Form aus der deutschen Patentanmeldung Nr. P 41 16 937.9 (HOE 91/F161) bekannt sind.

Die Verbindungen der Formel IV werden erfindungsgemäß durch Abspaltung der Oximschutzgruppe mittels equivalenter Mengen oder einem geringen Überschuß der oben genannten anorganischen Säuren oder organischen Sulfonsäuren hergestellt, wobei ein besonderer Vorteil des erfindungsgemäßen Verfahrens darin liegt, daß in einer Stufe die Schutzgruppenabspaltung und die Bildung der Endstufe in der gewünschten physiologisch verträglichen Salzform vonstatten geht.

Als Lösungsmittel eignen sich als organische Lösungsmittel Alkohole, Ester, Ether oder Ketone. Vorzugsweise werden Alkohole wie Methanol, Ethanol, n-Propanol, iso-Propanol oder die isomeren Butanole verwendet. Besonders bevorzugt sind diejenigen Lösungsmittel aus denen das Endprodukt der Formel I aus der Reaktionsmischung als Salz (x HX) ausfällt. Im speziellen erfindungsgemäßen Fall für die Verbindungen der Formel I mit R¹ = (S)-2,2-Dimethylpropionyloxy-1-ethyl und R² = Trityl oder Tetrahydropyranyl ist dies bevorzugt n-Propanol als Lösungsmittel und p-Toluolsulfonsäure als Schutzgruppen-abspaltendes Agens. Die Reaktionstemperaturen liegen zwischen +20° und +110°C, vorzugsweise zwischen +50° und +100°C. Besonders bevorzugt ist eine Temperatur zwischen +70° und +100°C. Nach Abkühlen der Reaktionsmischung, Absaugen und Trocknen wird das Endprodukt als Tosylatsalz rein erhalten.

Wenig geeignet erwiesen sich die Verfahren der Literatur. Die Tritylgruppe wird in der Cephalosporinchemie, z. B. in EP-A 355821, ausschließlich in stark saurer Lösung, z. B. in 90 % wässriger Ameisensäure oder in Trifluoressigsäure, abgespalten. Gemäß der Erfindung erfolgt die Schutzgruppenabspaltung und Salzbildung in einem Schritt. Die Endstufen bilden sich sowohl in hoher Ausbeute als auch in reiner Form.

Gegenstand der Erfindung ist weiterhin die Verbindung der Formel II' 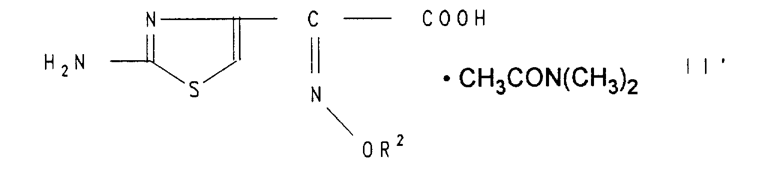 mit R² = Trityl, die als CH₃CON(CH₃)₂-Addukt vorliegt sowie ein neues Verfahren zur Herstellung dieser Verbindung der Formel II', dadurch gekennzeichnet, daß man 2-Aminothiazol-4-yl-2-hydroxyimino-essigsäure-ethylester mit Triphenylmethylchlorid und Kalium-tert.-butylat bei Raumtemperatur in inerten Lösungsmitteln umsetzt, den gebildeten Ethylester hydrolysiert und die erhaltene rohe Säure mit N,N-Dimethylacetamid bei Temperaturen zwischen +20° und +70° C behandelt.

Ein ähnliches Verfahren zur Herstellung von Verbindungen der Formel II mit R² = Trityl ist aus EP-A 355 821 bekannt. Die Nachteile des dort beschriebenen Verfahrens bestehen unter anderem in der Verwendung des gefährlichen Natriumhydrids, dessen Anwendung in größerem Maßstab aufwendige Vorsichtsmaßnahmen erfordert. Mit dem einfacher zu handhabendem Kalium-tert.-butylat als HCI-abspaltendem Agens verläuft die Tritylierung glatt bei Raumtemperatur in inerten Lösungsmitteln wie Ethern oder Estern, vorzugsweise in Tetrahydrofuran. Nach Verseifung wird die rohe O-Tritylcarbonsäure mit N,N-Dimethylacetamid bei Temperaturen zwischen +20° und +70° C behandelt, wobei sich das Addukt der Formel II' in hochreiner Form und in hoher Ausbeute bildet.

Die neue Verbindung der Formel II' mit R² = Trityl läßt sich in hoher Ausbeute mit den Verbindungen der Formel III zu den Verbindungen der Formel IV umsetzen.

Gegenstand der Erfindung ist weiterhin die Verbindung der Formel II" 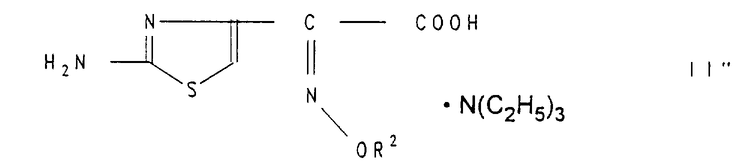 mit R² = 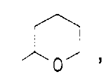 die als Triethylaminsalz vorliegt und die sich ebenfalls in hoher Ausbeute mit den Verbindungen der Formel III zu den Verbindungen der Formel IV umsetzen läßt.

Diese Umsetzung verläuft nach Aktivierung über das Säurechlorid oder das gemischte Anhydrid, z.B. mit Sulfosäuren, wie Methan-, Benzol-, p-Toluolsulfonsäure, oder einem Thioester, z.B. dem 2-Benzthiazolylthioester, 6-Ethoxy- oder 5-Chlor-2-benzthiazolylthioester. Bevorzugt ist die Aktivierung als Sulfonsäureanhydrid oder als 2-Benzthiazolylthioester der allgemeinen Formel V, worin R² = Trityl oder Tetrahydropyran-2-yl, R³ = Wasserstoff, Ethoxy oder Chlor bedeutet.

Die folgenden Ausführungsbeispiele für die nach dem erfindungsgemäßen Verfahren herstellbaren Verbindungen dienen zur weiteren Erläuterung der Erfindung, schränken sie jedoch nicht darauf ein.

Abkürzungen:
- THP: Tetrahydropyran
- DMAA: N,N-Dimethylacetamid

### Beispiel 1

(Z)-2-(2-Aminothiazol-4-yl)-2-trityloxyiminoessigsäure. Addukt mit N,N-Dimethylacetamid.

Zur Suspension von 258.3 g (1.2 mol) (Z)-2-(2-Aminothiazol-4-yl)-2-hydroxyiminoessigsäureethylester in 2 l trockenem Tetrahydrofuran werden unter Rühren und Kühlen bei +2°C 148 g (1.32 mol) Kalium-tert.butylat auf einmal zugegeben. Unter Erwärmung auf +4°C verfärbt sich die Suspension nach rotbraun. Die Mischung erreicht ohne Kühlung nach 30 Minuten Raumtemperatur. Es wird noch 1 Stunde nachgerührt, dann auf 15°C gekühlt und 362.4 g (1.3 mol) Tritylchlorid zugegeben. Ohne Außenkühlung wird noch 5 Stunden nachgerührt, wobei nach ca. 2 Stunden eine maximale Innentemperatur von +39°C erreicht wird. Die Suspension wird sodann in ein Gemisch aus 1.5 l Eiswasser und 1.2 l Diisopropylether gegossen, 1/2 Stunde gerührt, über Nacht bei 5°C belassen, der Niederschlag abgesaugt und mit 1 l Wasser und 700 ml Diisopropylether portionsweise gewaschen. Zur Verseifung wird das noch feuchte Produkt mit einer Lösung von 67.4 g (1.2 mol) Kaliumhydroxid in 1 l Wasser und 1.2 l Ethanol 3 Stunden gekocht. Nach 2 Stunden liegt eine klare, dunkle Lösung vor. Es wird auf 60°C gekühlt, 1.2 l Essigester zugegeben, auf +10°C gekühlt und unter Rühren innerhalb 15 Minuten mit ca. 190 ml 6N Salzsäure bis zu einem pH-Wert von 4,0 versetzt. Es bildet sich ein kristalliner Niederschlag. Nach 16 Stunden bei 5°C wird abgesaugt, mit 1.5 l Wasser, dann 1.5 l Diisopropylether portionsweise gewaschen und i.Vak. bei 80°C getrocknet.

Die rohe Säure wird in ein Gemisch aus 600 ml Dimethylacetamid und 1.2 l Toluol eingetragen, 10 Minuten bei 65°C gerührt, 1 Stunde im Eisbad gekühlt, abgesaugt, mit 800 ml Toluol portionsweise gewaschen und i.Vak. bei 80°C getrocknet.
Ausbeute: 372 g hellgraue Kristalle (60% d. Th), Zers. 179 -181°C
¹H-NMR (DMSO-d₆, 270 MHz): δ = 1.96 (s, 3H, CH₃CO); 2.80 (s, 3H, NCH₃); 2.95 (s, 3H, NCH₃); 6.69 (s, 1H, Thiazol); 7.2 - 7.42 (m, 15H, Trityl)

### Beispiel 2

### (Z)-2-(2-Aminothiazol-4-yl)-2-(tetrahydropyran-2-yl)oxyiminoessigsäure-Triethylaminsalz

Zu 14.6 g (85 mmol) 2-Aminothiazol-4-yl-glyoxylsäure und 7.4 ml (53 mmol) Triethylamin in 250 ml Methanol werden 10.5 g (90 mmol) O-(Tetrahydropyran-2 2-yl)hydroxylamin portionsweise zugegeben. Die Suspension wird 1.5 Stunden bei Raumtemperatur gerührt. Es werden nochmals 5.2 ml (38 mmol) Triethylamin zugegeben, worauf eine hellgelbe Lösung entsteht. Man beläßt über Nacht bei Raumtemperatur, entfernt das Lösungsmittel i.Vak. und digeriert den zunächst öligen Rückstand mit Ether worauf Kristallisation eintritt. Es wird abgesaugt, mit Ether gewaschen und getrocknet.
Ausbeute: 30.2 g (96% d. Th), Zers. 141 - 144°C
¹H-NMR (DMSO-d₆, 270 MHz): δ = 1.16 (t, 9H, NEt₃); 1.3 - 1.8 (m, 6 THP-H); 2.98 (q, 6H, NEt₃); 3.4 und 3.88 (je 1m, 2 THP-H); 5.10 (s, 1 THP-H); 6.62 (s, 1H, Thiazol); 7.0 (s, 2H, NH₂)

### Beispiel 3

### (Z)-2-(2-Aminothiazol-4-yl)-2-trityloxyiminoessigsäure-2-benzthiazolylthioester

Eine Suspension von 125.9 g (480 mmol) Triphenylphosphin und 159.5 g (480 mmol) Bis(benzthiazol-2-yl)disulfid in 800 ml trockenem Dichlormethan wird 60 Minuten bei Raumtemperatur gerührt. Es wird auf 15°C gekühlt und 206.6 g (400 mmol) (Z)-2-(2-Aminothiazol-4-yl)-2-trityloxyiminoessigsäure-DMAA-Addukt auf einmal eingetragen. Unter Erwärmung auf 26°C entsteht eine gut rührbare Suspension. Es wird 50 Minuten bei Raumtemperatur gerührt, auf 10°C gekühlt und 40.5 g (400 mmol) Triethylamin innerhalb 25 Minuten zugetropft. Die Suspension wird noch 5Stunden bei Raumtemperatur gerührt, dann auf 5°C gekühlt, abgesaugt, zweimal mit je 30 ml Dichlormethan (10°C) und dreimal mit je 50 ml Diisopropylether gewaschen und i.Vak. bei 50°C getrocknet.
Ausbeute: 222.6 g (96% d. Th.), Zers. 187 - 189°C
Gehalt nach HPLC: 99.3%,
Nebenprodukt 0.3% 2-Mercaptobenzthiazol
¹H-NMR (DMSO-d₆, 270 MHz): δ = 6.84 (s, Thiazol-H); 7.22 - 7.42 (m, 15H, Trityl); 7.62 (2 arom. H); 8.12 und 8.28 (je 1 arom. H)

### Beispiel 4

### (Z)-2-(2-Aminothiazol-4-yl)-2-(tetrahydropyran-2-yl)-oxyiminoessigsäure-2-benzthiazolylthioester

59.0 g (225 mmol) Triphenylphosphin, 74.8 g (225 mmol) Bis(benzthiazol-2-yl)-disulfid und 64.1 g (172 mmol) (Z)-2-(2-Aminothiazol-4-yl)-2-(tetrahydropyran-2-yl)-oxyiminoessigsäure Triethylaminsalz in 630 ml Dichlormethan werden wie in Beispiel 3 beschrieben, umgesetzt. Nach 6 Stunden Rühren bei Raumtemperatur wird auf 5°C gekühlt, der Niederschlag abgesaugt und mit wenig kaltem Dichlormethan gewaschen.
Ausbeute: 53.7 g (72% d. Th.), Zers. 158 - 161°C
¹H-NMR (CDCl₃, 270 MHz): δ = 1.4 - 2.0 (m, 6 THP-H); 3.6 - 4.0 (m, 2 THP-H); 5.50 (s, 1 THP-H); 6.62 (s, 2H, NH₂); 6.85 (s, Thiazol-H); 7.50 (2 arom. H); 7.94 und 8.10 (je 1 arom. H)

### Beispiel 5

### 7- [2-(2-Aminothiazol-4-yl)-2-(Z)-hydroxyiminoacetamido]-3-methoxy-methyl-3-cephem-4-carbonsäure-1-(1S)[(2,2-dimethylpropionyloxy)ethyl]ester p-Toluolsulfonat

57.8 g (100 mmol) (Z)-2-(2-Aminothiazol-4-yl)-2-trityloxyiminoessigsäure-2-benzthiazolylthioester und 33.5 g (90 mmol) 7-Amino-3-methoxymethyl-3-cephem-4-carbonsäure-1-(1S)[(2,2-dimethylpropionyloxy)]ethylester (S : R = 97 : 3) werden in 450 ml N,N-Dimethylformamid 45 Minuten bei Raumtemperatur gerührt (24 - 28°C, schwach exotherm). Die Lösung läßt man in 2.6 1 halbkonzentrierte NaCl einlaufen, rührt 10 Minuten, saugt den gebildeten Niederschlag ab, wäscht dreimal mit je 100 ml Wasser und trocknet i.Vak.

### Tritylspaltung und Tosylatbildung

Das erhaltene Gemisch aus der tritylgeschützten Titelverbindung und 2-Mercaptobenzthiazol wird zusammen mit 21.3 g (112 mmol) p-Toluolsulfonsäuremonohydrat in 450 ml n-Propanol 30 Minuten auf 85 - 90°C erhitzt. Nach 5 Minuten beginnt die Abscheidung eines kristallinen Niederschlags. Die Suspension wird auf 15°C gekühlt, abgesaugt, dreimal mit je 25 ml n-Propanol und mit Diisopropylether gewaschen und i.Vak. 1 Stunde bei 50°C getrocknet.
Ausbeute: 55.4 g (86% d. Th.), farblose Kristalle, HPLC 100% Gehalt
¹H-NMR (DMSO-d₆, 270 MHz): δ = 1.15 (s, 9H, C(CH₃)₃; 1.48 (d, 3H, CHCH₃); 2.29 (s, 3H, Tosyl-CH₃); 3.20 (s, 3H, OCH₃); 3.59 (AB, 2H, SCH₂); 4.14 (s, 2H, CH₂O); 5.24 (d, 1H, H-6); 5.85 (dd, 1H, H-7); 6.82 (s, 1H, Thiazol-H); 6.87 (q, 1H, CHCH₃); 7.11 und 7.48 (je 2H, AA'XX', Tosyl-H); 8.0 - 9.0 (br, 3H, NH₃), 9.67 (d, 1H, Amid-NH); 12.04 (s, 1H, NOH)

### Tritylspaltung mit Ameisensäure zum Vergleich

Das aus einem gleichartigen 1/10 Ansatz erhaltene Gemisch aus der tritylgeschützten Titelverbindung und 2-Mercaptobenzthiazol wird in 60 ml 80% Ameisensäure 1 Stunde bei Raumtemperatur gerührt. Das gebildete Triphenylcarbinol wird abfiltriert, mit 10 ml 80% HCOOH gewaschen, das Filtrat in 500 ml Eiswasser eingerührt und bei 5 - 10°C durch Zugabe von 60 ml konz. wässrigem NH₃ auf pH 4.0 gestellt. Der Niederschlag wird abgesaugt, mit Wasser gewaschen und getrocknet.

### Tosylatbildung:

Das amorphe Produkt wird zusammen mit 1.71 g (9 mmol) p-Toluolsulfonsäurehydrat in 20 ml n-Propanol gelöst. Der gebildete Niederschlag wird nach 2 Stunden Stehen bei 15°C abgesaugt, dreimal mit je 4 ml n-Propanol und mit Diisopropylether gewaschen. Nach dem Trocknen erhält man 5.25 g (73.5%) der Titelverbindung. Das NMR-Spektrum ist mit dem der oben erhaltenen Verbindung identisch. Die Reinheit nach HPLC beträgt 95% im Vergleich zur obigen Verbindung.

### Beispiel 6

### Verbindung des Beispiels 5 aus dem THP-geschützten Thioester

5.2 g (11.9 mmol) Aktivester aus Beispiel 4 und 4.03 g (10.8 mmol) 7-Amino-3-methoxymethyl-3-cephem-4-carbonsäure-1-(1S)[2,2-dimethylpropionyloxy]ethylester (S : R = 98 : 2) werden in 54 ml DMF wie in Beispiel 5 umgesetzt. Das trockene Rohprodukt wird mit 4.1 g (21.6 mmol) p-Toluolsulfonsäuremonohydrat in 84 ml n-Propanol bei 80 - 85°C in das Tosylat überführt.
Ausbeute: 6.4 g (83% d. Th.), farblose Kristalle

Die Verbindung ist in allen Eigenschaften mit der aus Beispiel 5 identisch.

### Beispiel 7

### Verbindung des Beispiels 5

### Sulfonsäureanhydridverfahren mit der Tritylsäure aus Beispiel 1

Zur Suspension von 27.4 g (53 mmol) (Z)-2-(Aminothiazol-4-yl)-2-trityloxyiminoessigsäure-DMAA-Addukt (Beispiel 1) in 90 ml N,N-Dimethylacetamid und 10 ml trockenem Aceton werden unter Argon bei 15°C 5.37 g (53 mmol) Triethylamin zugegeben. Die Suspension wird dann auf -20°C gekühlt und 9.91 g (52 mmol) p-Toluolsulfonsäurechlorid zugegeben. Es wird 2 1/4 Stunden bei -14° bis-10°C nachgerührt. Die gebildete, fast klare gelbliche Lösung wird sodann auf -35°C gekühlt und eine Lösung von 14.9 g (40 mmol) 7-Amino-3-methoxymethyl-3 -cephem-4-carbonsäure-1-(1S)[(2,2-dimethylpropionyloxy)ethyl]ester (S R = 97 : 3) in 30 ml DMAA während 15 Minuten zugetropft. Es wird 45 Minuten bei -25°C nachgerührt. Die Reaktionslösung wird in ein Gemisch aus 300 g Eis und 300 ml gesättigte NaHCO₃-Lösung eingetragen, die Suspension 2 Stunden gerührt, abgesaugt und dreimal mit je 200 ml Wasser gewaschen. Der feuchte Niederschlag wird in 600 ml Essigester gelöst, dreimal mit je 200 ml halbkonz. NaHCO₃-Lösung, dreimal 100 ml Wasser und zweimal 100 ml gesättigte NaCl-Lösung gewaschen, mit MgSO₄ getrocknet und abgedampft.

### Tritylspaltung und Tosylatbildung

Der amorphe Rückstand wird nach Zugabe von 7.62 g (40 mmol) p-Toluolsulfonsäurehydrat und 200 ml n-Propanol 30 Minuten auf 85 - 90° erhitzt. Es bildet sich ein kristalliner Niederschlag, der nach Abkühlen auf 15°C abgesaugt, dreimal mit je 10 ml n-Propanol und mit Diisopropylether gewaschen wird. Nach Trocknen i.Vak. bei 50°C erhält man 22.0 g (77% d. Th) farblose Kristalle. HPLC: 99% im Vergleich zum Produkt aus Beispiel 5. Das NMR-Spektrum ist identisch mit dem des Produkts aus Beispiel 5.

### Tritylspaltung mit Ameisensäure zum Vergleich

Der vorstehende Ansatz wird in halber Größe mit 7.45 g (20 mmol) 7-Amino-3-methoxymethyl-3-cephem-4-carbonsäure-1-(1S)[2,2-dimethylpropionyloxy)ethyl] ester analog ausgeführt. Die amorphe tritylgeschützte Titelverbindung wird in 120 ml 80% wässriger Ameisensäure gelöst und 1 Stunde bei Raumtemperatur gerührt. Das ausgefallene Triphenylcarbinol wird abgesaugt und mit 20 ml 80% HCOOH gewaschen. Das Filtrat wird in 700 ml Eiswasser eingerührt und bei 10°C durch Zugabe von 120 ml konz. NH₃ auf pH 4.0 gebracht. Der Niederschlag wird abgesaugt, mit Wasser gewaschen und über P₂O₅ bei 1 Torr getrocknet.

### Tosylatbildung:

Das erhaltene amorphe Produkt (9.3 g) wird in 37 ml n-Propanol gelöst und mit einer Lösung von 3.0 g (16 mmol) p-Toluolsulfonsäuremonohydrat in 8 ml n-Propanol versetzt. Man beläßt über Nacht im Kühlschrank, saugt ab, wäscht zweimal mit je 5 ml n-Propanol, dann mit Diisopropylether und trocknet i.Vak. über P₂O₅.
Ausbeute: 9.85 g (69% d. Th.)
Reinheit nach HPLC 96% im Vergleich zum Produkt aus Beispiel 5.
Das NMR-Spektrum ist identisch mit dem des Produkts aus Beispiel 5.

### Beispiel 8

### Verbindung des Beispiels 5

### Sulfonsäureanhydridverfahren mit der THP-Säure aus Beispiel 2

Eine Lösung von 5.35 g (14.36 mmol) (Z)-2-(2-Aminothiazol-4-yl)-2-(tetrahydropyran-2-yl)oxyiminoessigsäure-Triethylaminsalz in 18 ml N,N-Dimethylacetamid und 2 ml trockenem Aceton wird auf -20°C gekühlt und eine Lösung von 2.61 g (13.7 mmol) p-Toluolsulfonsäurechlorid in 5 ml DMAA zugegeben. Es wird 1 1/2 Stunden bei -15° bis -10°C gerührt, dann auf -30°C gekühlt und eine Lösung von 3.73 g (10 mmol) 7-Amino-3-methoxymethyl- 3-cephem-4-carbonsäure- 1-(1S)-[(2,2-dimethylpropionyloxy)]ethylester in 9 ml DMAA in 10 Minuten zugetropft. Die Lösung wird 45 Minuten bei -25°C nachgerührt. Sodann wird in ein Gemisch aus 100 g Eis und 100 ml gesättigte NaHCO₃-Lösung eingerührt, 1 Stunde nachgerührt, der ausgefallene Niederschlag abgesaugt, mit Wasser gewaschen, in 200 ml Essigester gelöst und die Lösung mit 50ml gesättigter NaHCO₃-Lösung, mit Wasser und zweimal mit je 50 ml gesättigter NaCl-Lösung gewaschen. Nach Trocknen mit MgSO₄ wird abgedampft.

### THP-Spaltung und Tosylatbildung

Der amorphe Rückstand (7.3 g) wird mit 2.85 g (15 mmol) p-Toluolsulfonsäurehydrat in 50 ml n-Propanol gelöst und 25 Minuten auf 90°C erhitzt. Nach 2 Minuten beginnt die Abscheidung eines kristallinen Niederschlags. Die Suspension wird auf 15°C gekühlt, abgesaugt, dreimal mit je 8 ml n-Propanol und mit Diisopropylether gewaschen und i.Vak. bei 50°C 1 Stunde getrocknet.
Ausbeute: 5.3 g (74% d. Th.) farblose Kristalle
Reinheit nach HPLC: 99% im Vergleich zum Produkt aus Beispiel 5.
Das NMR-Spektrum ist identisch mit dem der Verbindung aus Beispiel 5.

### Beispiel 9

### 7-[2-(2-Aminothiazol-4-yl)-2-(Z)-hydroxyiminoacetamido]-3-methoxymethyl-3-cephem-4-carbonsäure-1-(1R,S) [(isopropyloxycarbonyloxy)ethyl]ester p-Toluolsulfonat

905 mg (2,42 mmol) 7-Amino-3-methoxymethyl-3-cephem-4-carbonsäure-1-(1R,S)[(isopropyloxycarbonyloxy)ethyl]ester und 1,5 g (2,6 mmol) (Z)-2-(2-Aminothiazol-4-yl)-2-trityloxyiminoessigsäure-2-benzthiazolylthioester werden in 10 ml N,N-Dimethylformamid analog Beispiel 5 umgesetzt. Die Tritylgeschützte Titelverbindung wird mit 475 mg (2,5 mmol) p-Toluolsulfonsäurehydrat in 10 ml n-Propanol 25 Minuten bei 90° C erhitzt. Nachdem Abkühlen Kristallisieren 900 mg (52 % d. Th.) der Titelverbindung.
Wird analog Beispiel 5 zunächst mit Ameisensäure die Tritylgruppe abgespalten und sodann das Tosylal gebildet, so beträgt die Ausbeute 231 mg (13 % d. Th.).
¹H-NMR(DMSO-d₆; 270 MHz): δ = 1,24 (2d, 6H, CH(CH₃)₂); 1,50 (d, 3H, CHCH₃); 2,29 (s, 3H, Tosyl-CH₃); 3,21 (s, 3H, OCH₃); 3,60 (AB, 2H, SCH₂); 4,17 (s, 2H, CH₂O); 4,80 (m, 1H, CH(CH₃)₂); 5,84 (dd, 1H, H-7); 6,80 (q, 1H, CHCH₃; 6,83 (s, 1H, Thiazol-H); 7,12 und 7,48 (je 2H, AA'XX', Tosyl-H); 9,68 (d, 1H, Amid-NH); 12,12 (s, 1H, NOH).

### Beispiel 10

### (Z)-2-(2-Aminothiazol-4-yl)-2-trityloxyiminoessigsäure-6-ethoxy-2-benzthiazolylthioester

Eine Suspension von 10,5 g (40 mmol) Triphenylphosphin und 16,8 g (40 mmol) Bis(6-Ethoxy-benzthiazol-2-yl)disulfid in 70 ml trockenem Dichlormethan wird 35 Minuten bei Raumtemperatur gerührt. Bei 12°C werden 17,2 g (33,3 mmol) (Z)-2-(2-Aminothiazol-4-yl)-2-trityloxyiminoessigsäure-DMAA-Addukt zugegeben. Die Suspension wird 45 Minuten gerührt, auf 12°C gekühlt, 3,36 g (33,3 mmol) Triethylamin zugegeben und 4 Stunden bei Raumtemperatur gerührt. Es wird abgesaugt, portionsweise mit 150 ml Dichlormethan und 150 ml Diisopropylether gewaschen und i.Vak. getrocknet.
Ausbeute: 20,6 g (quantitativ), Zers. 218°C
¹H-NMR(DMSO-d₆; 200 MHz): δ = 1,41 (t, 3H, CH₂CH₃); 4,16 (q, 2H, CH₂CH₃); 6,82 (s, Thiazol-H); 7,18 - 7,42 (m, 16 arom.H); 7,80 und 8,00 (je 1 arom.H).

### Beispiel 11

### (Z)-2-(2-Aminothiazol-4-yl)-2-trityloxyiminoessigsäure-5-chlor-2-benzthiazolylthioester

2,6 g (10 mmol) Triphenylphosphin, 4,0 g (10 mmol) Bis (5-Chlor-benzthiazol-2-yl)disulfid, 4,3g (8,35 mmol) (Z) -2-(2-Aminothiazol-4-yl)-2-trityloxyiminoessigsäure-DMAA-Addukt und 0,84 g (8,35 mmol) Triethylamin werden in 20 ml Dichlormethan wie in Beispiel 10 beschrieben umgesetzt. Die Suspension wird 20 Stunden bei Raumtemperatur gerührt, der Niederschlag abgesaugt, mit 40 ml Dichlormethan und 40 ml Diisopropylether gewaschen und i.Vak. getrocknet.
Ausbeute: 5,0 g (quantitativ), Zers. 192°C
¹H-NMR(DMSO-d₆; 200 MHz): δ = 6,86 (s, Thiazol-H); 7,20 - 7,44 (m, 15 arom.H); 7,65, 8,20 und 8,32 (je 1 arom.H).

### Beispiel 12

### Verbindung des Beispiels 5 aus dem Aktivester von Beispiel 10.

3,12 g (5 mmol) (Z) -2-(2-Aminothiazol-4-yl)-2-trityloxyiminoessigsäure-6-ethoxy-2-benzthiazolylthioester und 1,9 g (5,1 mmol) 7-Amino-3-methoxymethyl-3-cephem-4-carbonsäure-1-(1S) [(2,2-dimethylpropionyloxy)ethyl]ester werden in 40 ml N,N-Dimethylformamid 18 Stunden bei Raumtemperatur gerührt. Die erhaltene klare gelbe Lösung läßt man in 300 ml halbkonzentrierte Kochsalzlösung einlaufen, saugt den Niederschlag ab, wäscht viermal mit je 20 ml Wasser und trocknet i.Vak. Ausbeute: 4,76 g (95,6% d.Th)
Das erhaltene Gemisch aus der tritylgeschützten Titelverbindung und 6-Ethoxy-2-mercaptobenzthiazol wird zusammen mit 1,05 g (5,5 mmol) p-Toluolsulfonsäuremonohydrat in 25 ml n-Propanol 30 Minuten auf 90°C erhitzt. Nach 10 Minuten beginnt die Ausscheidung eines Niederschlags. Nach Erreichen von 30°C wird abgesaugt, dreimal mit je 5 ml n-Propanol und mit Diisopropyläther gewaschen. Nach Trocknen i. Vak. erhält man 2,60 g farblose Kristalle (72,8% d.Th.). Die Verbindung ist in allen Eigenschaften mit der aus Beispiel 5 identisch.

### Beispiel 13

### Verbindung des Beispiels 5 aus dem Aktivester von Beispiel 11

3,07 g (5 mmol) (Z) -2-(2-Aminothiazol-4-yl)-2-trityloxyiminoessigsäure-5-chlor-2-benzthiazolylthioester und 1,9 g (5,1 mmol) 7-Amino-3-methoxymethyl-3-cephem-4-carbonsäure-1-(1S) [(2,2-dimethylpropionyloxy)ethyl]ester werden in 40 ml N,N-Dimethylformamid 2 Stunden bei Raumtemperatur gerührt. Die Lösung wird wie in Beispiel 12 beschrieben, aufgearbeitet, das Gemisch aus tritylgeschützter Titelverbindung und 5-Chlor-2-mercaptobenzthiazol mit 1,05 g (5,5 mmol) p-Toluolsulfonsäuremonohydrat in 25 ml n-Propanol 30 Minuten bei 90°C gerührt. Der gebildete Niederschlag wird nach Abkühlen auf 15°C abgesaugt, mit n-Propanol und Diisopropyläther gewaschen und i.Vak. getrocknet.

Ausbeute: 2,73 g (76,5% d.Th.) farblose Kristalle. Die Verbindung ist in allen Eigenschaften mit der aus Beispiel 5 identisch.

## Patentansprüche

1. Verfahren zur Herstellung einer Verbindung der Formel I, 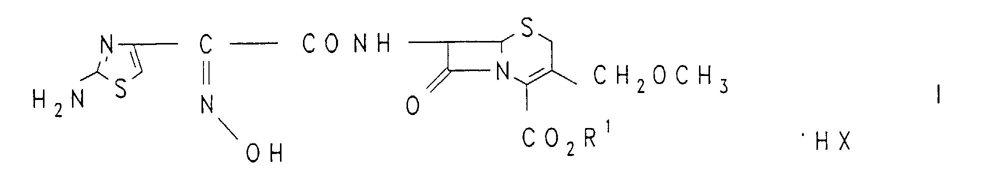 worin R¹ C₁-C₅-Alkanoyloxy-C₁-C₃-alkyl C₁-C₅-Alkoxycarbonyloxy-C₁-C₃-alkyl und H X eine anorganische oder organische Säure ausgewählt aus: HCI, HBr oder H₂SO₄ und Methan-, Ethan-, Benzol-, p-Toluol-, p-Chlorobenzol- oder 2,4-Dichlorobenzolsulfonsäure, bedeutet, und worin die Hydroxyiminogruppe in der syn-Form vorliegt, umfassend, daß man eine Verbindung der Formel II 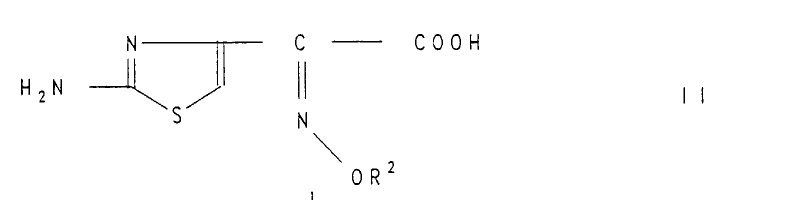 worin
R² eine durch saure Hydrolyse abspaltbare Schutzgruppe bedeutet, mit einem Bis(benzthiazol-2-yl)disulfid der Formel VI 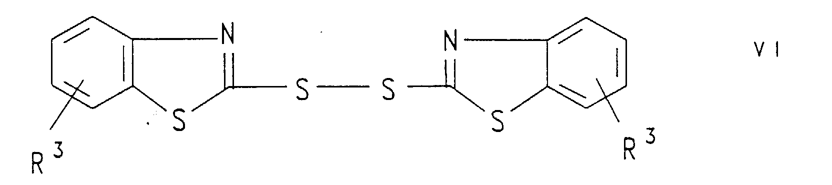 worin R³ Wasserstoff, C₁-C₆-Alkyl, C₂-C₆-Alkenyl, C₃-C₆-Alkinyl, C₁-C₆-Alkyloxy, C₂-C₆-Alkenyloxy, C₃-C₆-Alkinyloxy, Hydroxy, Acetoxy, Halogen, Nitro, Amino, Carboxy oder die Sulfogruppe bedeutet, und Triphenylphosphin in einem inerten Lösungsmittel, in Gegenwart eines tertiären Amins, zu einer Verbindung der Formel V 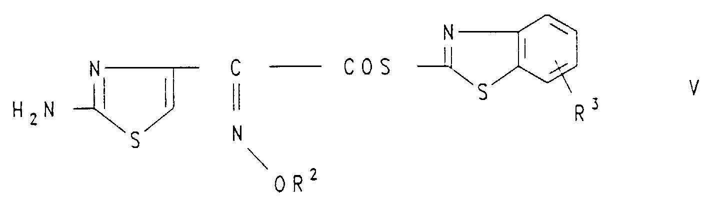 worin
R² und R³ die obengenannten Bedeutungen haben, reagieren läßt, anschließend diese Verbindung mit einem 7-Amino-ceph-3-em-4-carbonsäureester der Formel III 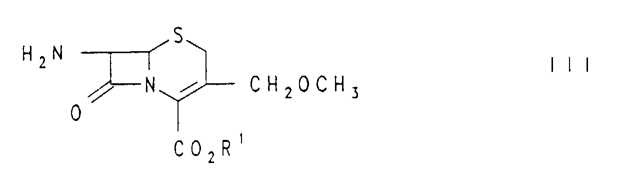 in der R¹ die obengenannte Bedeutung hat, in inerten organischen oder dipolar aprotischen Lösungsmitteln bei Temperaturen zwischen 0° und +80° C umsetzt und die Oxim-geschützte Verbindung der Formel IV, 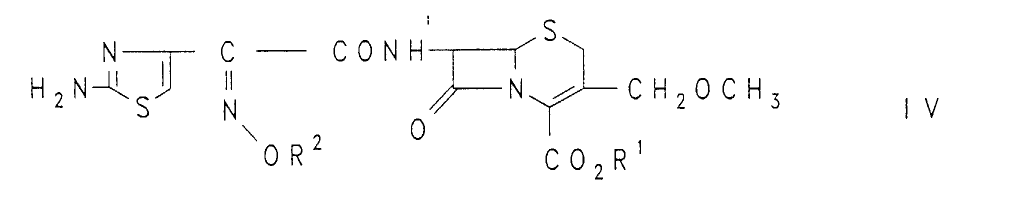 in der R¹ und R² die oben genannten Bedeutungen besitzen und worin die geschützte Oximgruppe in der syn-Form vorliegt bildete, dadurch gekennzeichnet, daß die Verbindung der Formel IV mit anorganischen Säuren oder mit aliphatischen oder aromatischen Sulfonsäuren ausgewählt aus: HCI, HBr oder H₂SO₄ und Methan-, Ethan-, Benzol-, p-Toluol-, p-Chlorobenzol- oder 2,4-Dichlorobenzolsulfonsäure, in organischen Lösungsmitteln ausgewählt aus: Alkohole, Ester, Ether und Ketone bei Temperaturen zwischen +20° und +110°C unter Bildung der Verbindung der Formel I in einem Schritt behandelt wird und, daß das Ausgangsprodukt der Formel II nicht NH₂ geschützt ist.

2. Verfahren zur Herstellung einer Verbindung der Formel I gemäß Anspruch 1, dadurch gekennzeichnet, daß
R¹ Acetoxymethyl, Propionyloxymethyl, Isopropionyloxymethyl, N-Butyryloxymethyl, Isobutyryloxymethyl, 2,2-Dimethylpropionyloxymethyl, Isovaleryloxymethyl, 1-Acetoxy-1-ethyl, 1-Acetoxy-1-propyl, 2,2-Dimethylpropionyloxy-1-ethyl, -1-ethyl, 1-Methoxycarbonyloxy-1-ethyl, 1-Ethoxycarbonyloxy-1-ethyl, 1-Isopropoxycarbonyloxyethyl oder Methoxycarbonyloxymethyl;
R² C(C₆H₅)₃, Tetrahydropyranyl oder 2-Methoxy-2-propyl;
R³ Wasserstoff, Methyl, Ethyl, Propyl, Methoxy, Ethoxy, Propoxy, Isopropoxy oder Chlor
und
X Cl⁻, Br⁻, HSO₄⁻, CH₃SO₃⁻, C₂H₅SO₃⁻, C₆H₅SO₃⁻, p-CH₃-C₆H₄-SO₃⁻ oder p-Cl-C₆H₄-SO₃⁻ bedeuten.

3. Verfahren zur Herstellung einer Verbindung der Formel I gemäß den Ansprüchen 1 oder 2, dadurch gekennzeichnet, daß R¹ 2,2-Dimethylpropionyloxy-1-ethyl in der (R)- oder (S)-Form bedeutet.

4. Verfahren zur Herstellung einer Verbindung der Formel I gemäß Anspruch 3, dadurch gekennzeichnet, daß R¹ 2,2-Dimethylpropionyloxy-1-ethyl in der (S)-Form bedeutet.

5. Verbindung der Formel II', 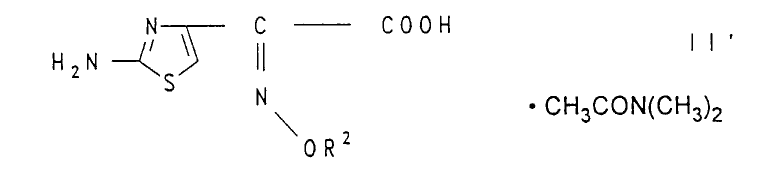 worin R² = C(C₆H₅)₃ bedeutet.

6. Verbindung der Formel II" 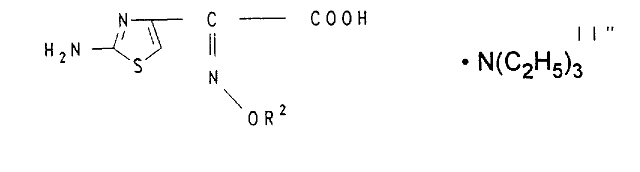 worin R² = 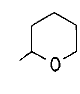 bedeutet.

7. Verfahren zur Herstellung einer Verbindung der Formel II' gemäß Anspruch 5, dadurch gekennzeichnet, daß man 2-Aminothiazol-4-yl-2-hydroxyiminoessigsäure-ethylester mit Triphenylmethylchlorid und Kalium-tert.-butylat bei Raumtemperatur in inerten Lösungsmitteln umsetzt, den gebildeten Ethylester hydrolysiert und die erhaltene rohe Säure mit N,N-Dimethylacetamid bei Temperaturen zwischen +20° und +70° C behandelt.

8. Verwendung der Verbindung der Formel II' gemäß Anspruch 5 bei der Herstellung einer Verbindung der Formel IV, mit R² = C(C₆H₅)₃.

9. Verwendung der Verbindung der Formel II" gemäß Anspruch 6 bei der Herstellung einer Verbindung der Formel IV mit R²=

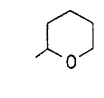

## Claims

1. A process for the preparation of a compound of the Formula I, 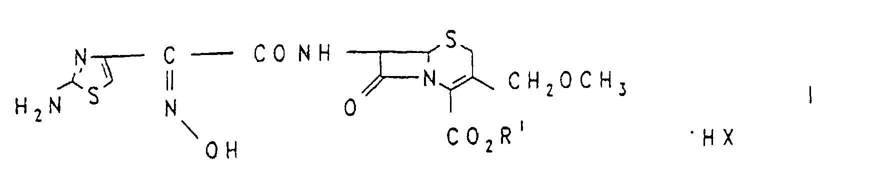 wherein R¹ is (C₁-C₅)alkanoyloxy(C₁-C₃)alkyl or (C₁-C₅)alkoxycarbonyloxy(C₁-C₃)alkyl and H X is an inorganic or organic acid selected from: HCI, HBr or H₂SO₄ and methane sulfonic acid, ethane sulfonic acid, benzenesulfonic acid, p-toluenesulfonic acid, p-chlorobenzol-sulfonicacid or 2,4-dichlorobenzenesulfonic acid, and wherein the hydroxyimino group is in the syn form, which comprises allowing a compound of the formula II 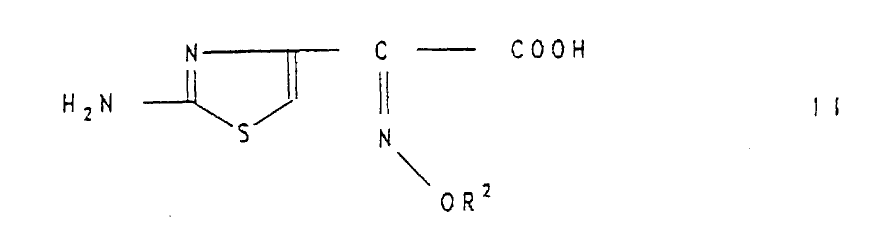 wherein R² is a protective group which is cleavable by acid hydrolysis, to react with a bis(benzthiazole-2-yl)disulfide of the formula VI 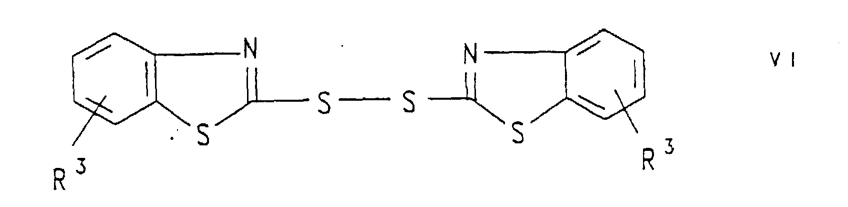 wherein R³ is hydrogen, (C₁-C₆)alkyl, (C₁-C₆)alkenyl, (C₁-C₆)alkinyl, (C₁-C₆)alkyloxy, (C₂-C₆)alkenyloxy, (C₃-C₆)alkinyloxy, hydroxy, acetoxy, halogen, nitro, amino, carboxy or the sulfo group, and triphenylphosphine in an inert solvent, in the presence of a tertiary amine, to afford a compound of the formula V 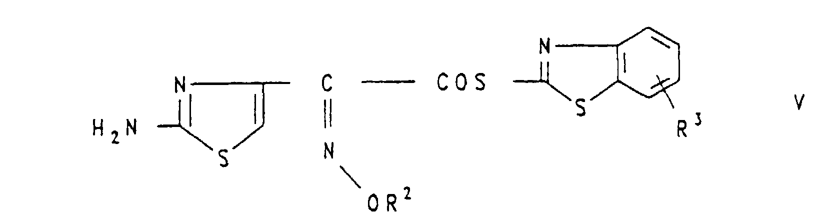 in which R² and R³ are as defined above, said compound being subsequently reacted with a 7-amino-ceph-3-em-4-carbonic acid ester of the formula III 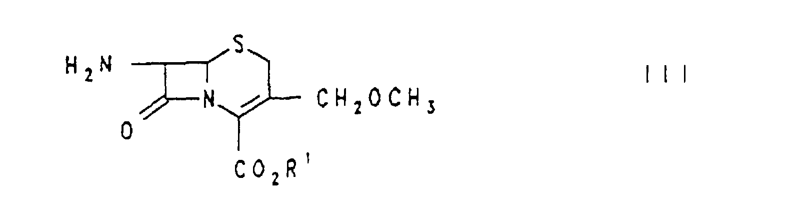 in which R¹ is as defined above, in inert organic or dipolar aprotic solvents at temperatures between 0°C and +80°C to afford the oxime-protected compound of the formula IV, 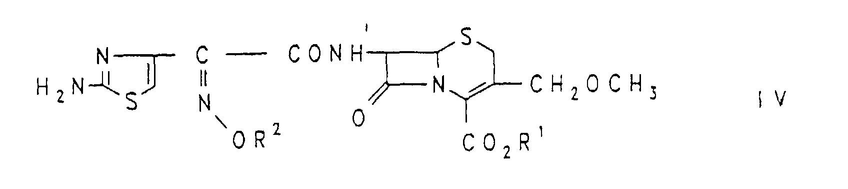 in which R¹ and R² are as defined above and wherein the protected oxime group is in the syn form, characterised in that the compound of the formula IV is treated with inorganic acids or with aliphatic or aromatic sulfonic acids selected from: HCl, HBr or H₂SO₄ and methane sulfonic acid, ethane sulfonic acid, benzenesulfonic acid, p-toluenesulfonic acid, p-chlorobenzolsulfonicacid or 2,4-dichlorobenzenesulfonic acid, in organic solvents selected from: alcohols, esters, ethers and ketones at temperatures betwenn +20°C and +110°C to afford the compound of the formula I in one step, and in that the initial product of the formula II is not NH₂-protected.

2. A process for the preparation of a compound of the Formula I as defined in claim 1, characterised in that
R¹ is acetoxymethyl, propionyloxymethyl, isopropionyloxymethyl, N-butyryloxymethyl, isobutyryloxymethyl, 2,2-dimethylpropionyloxymethyl, isovaleryloxymethyl, 1-acetoxy-1-ethyl, 1-acetoxy-1-propyl, 2,2-dimethylpropionyloxy-1-ethyl, 1 -methoxycarbonyloxy-1-ethyl, 1-ethoxycarbonyloxy-1-ethyl, 1-isopropoxycarbonyloxyethyl or methoxycarbonyloxymethyl ;
R² is C(C₆H₅)₃, tetrahydropyranyl or 2-methoxy-2-propyl;
R³ is hydrogen, methyl, ethyl, propyl, methoxy, ethoxy, propoxy, isopropoxy or chlorine; and
X is CI⁻, Br⁻, HSO₄⁻, CH₃SO₃⁻, C₂H₅SO₃⁻, C₆H_{S}SO₃⁻ or p-CI-C₆H₄-SO₃-

3. A process for the preparation of a compound of the Formula I as defined in claims 1 or 2, characterised in that R¹ is 2,2-dimethylpropionyloxy-1-ethyl in the (R) or (S) form.

4. A process for the preparation of a compound of the formula I as defined in claim 3, characterised in that R¹ is 2,2-dimethylpropionyloxy-1-ethyl in the (S) form.

5. A compound of the formula II' 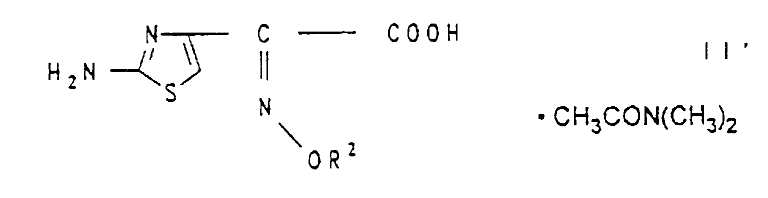 wherein R² is C(C₆H₅)₃.

6. A compound of the formula II" 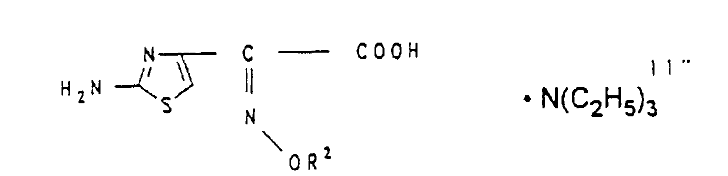 wherein R² is

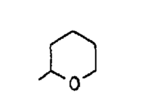

7. A process for the preparation of a compound of the formula II' as defined in claim 5, characterised in that 2-aminothiazole-4-yl-2-hydroxyiminoacetic acid ethyl ester is reacted with triphenylmethyl chloride and potassium-tert-butylate at ambient temperature in inert solvents, the resulting ethyl ester is hydrolysed and the generated raw acid is treated with N,N-dimethyl acetamide at temperatures between +20°C and +70°C.

8. Use of the compound of the formula II' as defined in claim 5 for the preparation of a compound of the formula IV, wherein R² is C(C₆H₅)₃.

9. Use of the compound of the formula II" as defined in claim 6 for the preparation of a compound of the formula IV, wherein R² is

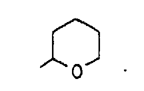

## Revendications

1. Procédé pour la préparation d'un composé de formule I 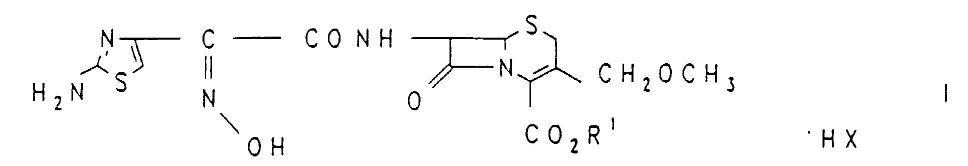 dans laquelle R¹ représente un groupe alcanoyloxy(C₁-C₅)-alkyle(C₁-C₃) ou alcoxy(C₁-C₅)carbonyloxy-alkyle(C₁-C₃) et HX représente un acide minéral ou organique choisi parmi: HCl, HBr ou H₂SO₄ et l'acide méthane-, éthane-, benzène-, p-toluène-, p-chlorobenzol- ou 2,4-dichlorobenzènesulfonique, et dans laquelle le groupe hydroxyimino se trouve sous la forme *syn*,
comprenant la mise en réaction d'un composé de formule II 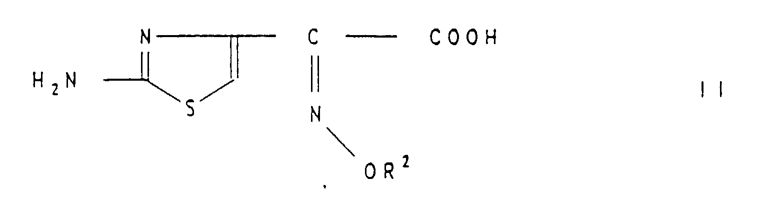 dans laquelle
R² représente un groupe protecteur pouvant être éliminé par hydrolyse acide,
avec un disulfure de bis(benzothiazol-2-yle) de formule VI 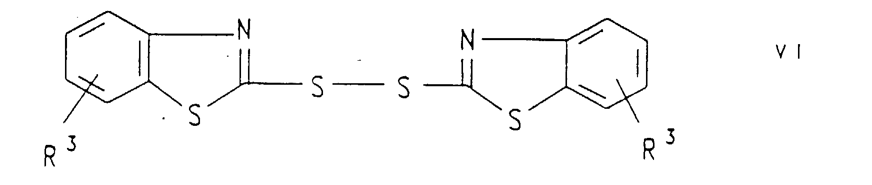 dans laquelle R³ représente un atome d'hydrogène ou d'halogène ou un groupe alkyle en C₁-C₆, alcényle en C₂-C₆, alcynyle en C₃-C₆, alkyloxy en C₁-C₆, alcényloxy en C₂-C₆, alcynyloxy en C₃-C₆, hydroxyle, acétoxy, nitro, amino, carboxyle ou le groupe sulfo, et de la triphénylphosphine dans un solvant inerte, en présence d'une amine tertiaire, conduisant à un composé de formule V 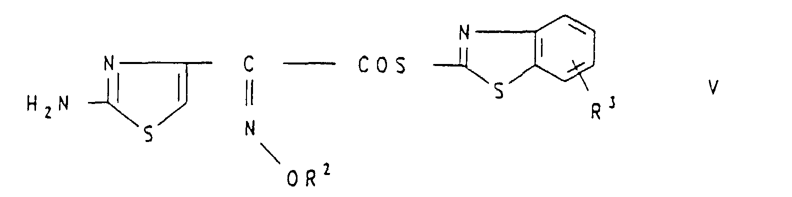 dans laquelle
R² et R³ ont les significations données plus haut, suivie de la mise en réaction de ce composé avec un ester d'acide 7-amino-céph-3-ème-4-carboxylique de formule III 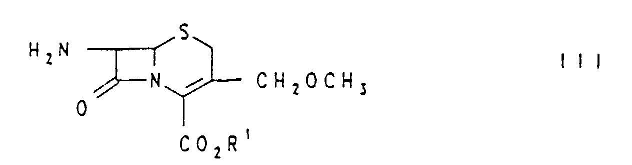 dans laquelle R¹ a la signification donnée plus haut, dans des solvants inertes organiques ou dipolaires aprotiques, à des températures comprises entre 0 et +80°C, avec formation du composé protégé sur la fonction oxime, de formule IV, 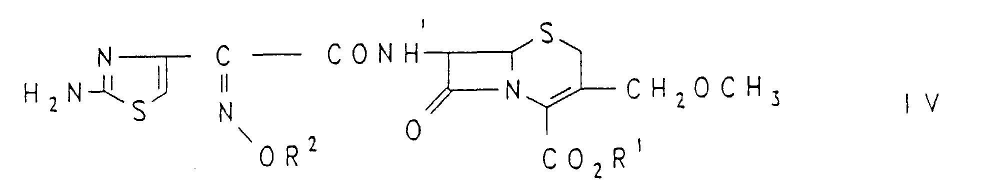 dans laquelle R¹ et R² ont les significations données plus haut et dans laquelle le groupe oxime protégé se trouve sous la forme *syn*,
caractérisé en ce que l'on traite le composé de formule IV par des acides minéraux ou par des acides sulfoniques aliphatiques ou aromatiques, choisis parmi:
HCl, HBr ou H₂SO₄ et l'acide méthane-, éthane-, benzène-, p-toluène-, p-chlorobenzol- ou 2,4-dichlorobenzènesulfonique, dans des solvants organiques choisis parmi: des alcools, des esters, des éthers et des cétones, à des températures comprises entre +20°C et 110°C, avec formation du composé de formule I en une étape, et en ce que le produit de départ de formule II n'est pas protégé sur NH₂.

2. Procédé pour la préparation d'un composé de formule I selon la revendication 1, caractérisé en ce que
R¹ représente le groupe acétoxyméthyle, propionyloxyméthyle, isopropionyloxyméthyle, N-butyryloxyméthyle, isobutyryloxyméthyle, 2,2-diméthylpropionyloxyméthyle, isovaléryloxyméthyle, 1-acétoxy-1-éthyle, 1-acétoxy-1-propyle, 2,2-diméthylpropionyloxy-1-éthyle, 1-méthoxycarbonyloxy-1-éthyle, 1-éthoxycarbonyloxy-1-éthyle, 1-isopropoxycarbonyloxyéthyle ou méthoxycarbonyloxyméthyle;
R² représente le groupe C(C₆H₅)₃, tétrahydropyrannyle ou 2-méthoxy-2-propyle;
R³ représente un atome d'hydrogène ou de chlore, ou le groupe méthyle, éthyle, propyle, méthoxy, éthoxy, propoxy ou isopropoxy
et
X représente Cl⁻, Br⁻, HSO₄⁻, CH₃SO₃⁻, C₂H₅SO₃⁻, C₆H₅SO₃⁻ ou p-CH₃-C₆H₄-SO₃⁻ ou p-Cl-C₆H₄-SO₃⁻

3. Procédé pour la préparation d'un composé de formule I selon la revendication 1 ou 2, caractérisé en ce que R¹ représente le groupe 2,2-diméthylpropionyloxy-1-éthyle sous la forme (R) ou (S).

4. Procédé pour la préparation d'un composé de formule I selon la revendication 3, caractérisé en ce que R¹ représente le groupe 2,2-diméthylpropionyloxy-1-éthyle sous la forme (S).

5. Composé de formule Il' 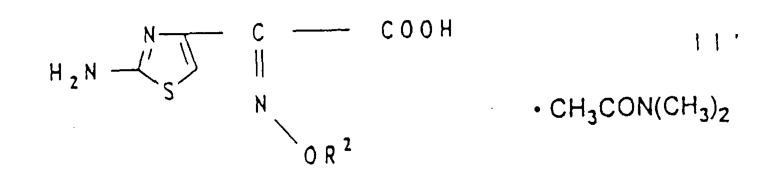 dans laquelle R² représente le groupe C(C₆H₅)₃.

6. Composé de formule II" 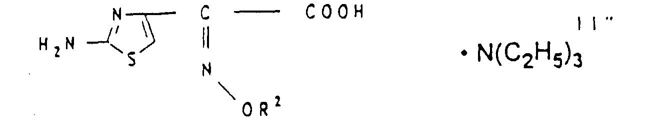 dans laquelle R² représente

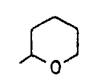

7. Procédé pour la préparation d'un composé de formule II' selon la revendication 5, caractérisé en ce que l'on fait réagir du 2-aminothiazol-4-yl-2-hydroxyiminoacétate d'éthyle avec du chlorure de triphénylméthyle et du tert-butylate de potassium, à la température ambiante, dans des solvants inertes, on hydrolyse l'ester d'éthyle formé et on traite l'acide brut obtenu par du N,N-diméthylacétamide à des températures comprises entre +20° et +70°C.

8. Utilisation du composé de formule II' selon la revendication 5, dans la préparation d'un composé de formule IV dans lequel R²= C(C₆H₅)₃.

9. Utilisation du composé de formule II' selon la revendication 6, dans la préparation d'un composé de formule IV dans lequel R²=
